(19) Europäisches Patentamt

European Patent Office

Office européen des brevets

(11) **EP 0 464 461 B1**

(12) **EUROPÄISCHE PATENTSCHRIFT**

(45) Veröffentlichungstag und Bekanntmachung des Hinweises auf die Patenterteilung:
**15.11.2000 Patentblatt 2000/46**

(51) Int. Cl.[7]: **C12N 15/52**, C12N 15/63, C12N 1/20, C12N 15/82, A01H 5/00, A01H 1/00

(21) Anmeldenummer: **91110002.2**

(22) Anmeldetag: **19.06.1991**

(54) **Stilbensynthase-Gene aus Weinrebe**

Stilbensynthase gene of grapevine

Gène de stilbensynthase de la vigne

(84) Benannte Vertragsstaaten:
**BE CH DE FR GB IT LI NL**

(30) Priorität: **29.06.1990 DE 4020648**
**08.03.1991 DE 4107396**

(43) Veröffentlichungstag der Anmeldung:
**08.01.1992 Patentblatt 1992/02**

(73) Patentinhaber: **BAYER AG**
**51368 Leverkusen (DE)**

(72) Erfinder:
• **Hain, Rüdiger, Dr.**
**W-4018 Langenfeld (DE)**
• **Reif, Hans-Jörg, Dr.**
**W-5000 Köln (DE)**
• **Stenzel, Klaus, Dr.**
**W-4000 Düsseldorf 13 (DE)**

(56) Entgegenhaltungen:
**EP-A- 0 309 862**

• **FEBS LETTERS Band 268, Nr. 1, 30. Juli 1990, Seiten 17-20, Amsterdam, NL; F. MELCHIOR et al.: "Grapevine stilbene synthase cDNA only slightly differing from chalcone synthase cDNA is expressed in E.coli into a catalytically active enzyme"**
• **idem**
• **EUROPEAN JOURNAL OF BIOCHEMISTRY Band 172, Nr. 1, 1988, Seiten 161-169, Berlin, DE; G. SCHROEDER et al.: "Molecular analysis of resveratrol synthase cDNA, genomic clones and relationship with chalcone synthase"**
• **CHEMICAL ABSTRACTS Band 113, Nr. 9, 27. August 1990, Seite 466, Zusammenfassung Nr. 75086t, Columbus, Ohio, US; T. LANZ et al.: "Differential regulation of genes for resveratrol synthase in cell cultures of Arachis hypogaea" & Planta. 1990, Band 181, Nr. 2, Seiten 169-175**

**Beschreibung**

Die vorliegende Erfindung betrifft die aus Weinreben isolierten Gene für Stilbensynthase und ihre Verwendung zur Transformation von Vektoren, Wirtsorganismen und Pflanzen sowie zur Erzeugung von Pflanzen, welche eine erhöhte Resistenz gegenüber Schädlingen aufweisen.

Der Begriff Stilbene beschreibt eine Gruppe von chemischen Substanzen, welche in Pflanzen vorkommen und als gemeinsame Grundstruktur das Stilbengerüst (trans-1,2-Diphenylethylen) enthalten. Dieses Grundgerüst kann durch die Addition weiterer Gruppen ergänzt werden. Zwei wichtige Stilbene sind das 3,5-Dihydroxy-stilben (Pinosylvin) und das 3,3',5-Trihydroxy-stilben (Resveratrol).

Stilbene wurden in bestimmten Bäumen (Angiospermen, Gymnospermen), jedoch auch in einigen krautigen Pflanzen gefunden (in Arten der Familien Myrtaceae, Vitaceae und Leguminosae). Stilbene sind toxisch für Schädlinge, insbesondere für Pilze, Bakterien, Viren und Insekten und sind geeignet, diese Schädlinge abzuwehren. Die Fähigkeit der Synthese dieser Substanzen durch die Pflanzen wird als wichtiger Abwehrmechanismus angesehen. Leider haben nur wenige Nutzpflanzen die Fähigkeit Stilbene zu bilden, bzw. in einem Maße zu erzeugen, welches ihnen eine ausreichende Resistenz gegen Schädlinge verleiht.

Die Verwendung von Stilbensynthase-Genen zur Erzeugung von Pflanzen mit einer erhöhten Schädlingsresistenz ist bereits aus der EP-A-0 309 862 bekannt. In dieser Veröffentlichung wird speziell ein Stilbensynthase-Gen aus Erdnußpflanzen (Arachis hypogea) beschrieben.

Es wurden nun neue Gene für Stilbensynthase ("Stilbensynthase-Gene") aus Weinrebe isoliert, welche in die Erbmasse (das Genom) von Pflanzen eingebaut werden können, die keine Stilbene oder nur unzureichend Stilbene erzeugen, wodurch eine erhöhte Resistenz dieser Pflanzen gegen Schädlinge hervorgerufen werden kann.

Überraschenderweise ergeben die neuen Stilbensynthase-Gene aus Wein wesentlich günstigere Schädlingsresistenzen in Pflanzen als das aus dem Stand der Technik vorbekannte Stilbensynthase-Gen aus Erdnuß.

Unter Stilbensynthase-Genen sollen Nukleinsäuren (DNA) verstanden werden, die nach ihrer Transkription in RNA und Translation in Protein (in einer geeigneten Umgebung) die Bildung eines Enzyms bewirken, welches die Eigenschaften einer Stilbensynthase (enzymatische Synthese von Stilben in einer geeigneten Umgebung) besitzt, wobei diese Nukleinsäuren aus ihrer natürlichen Umgebung isoliert vorliegen oder in einen Vektor integriert sind oder in einer prokaryontischen oder eukaryontischen DNA als "fremde" DNA oder als "zusätzliche" DNA enthalten sind.

Unter Stilbensynthase-Genen sollen auch solche Stilbensynthase-Gene verstanden werden, die an ihrem Anfang und/oder Ende noch DNA-Sequenzen enthalten, die die Funktion der Gene nicht oder nicht wesentlich behindern, Diese auch als "Gen-Einheiten" bezeichneten DNA-Sequenzen entstehen, z.B. durch das Herausschneiden mit Restriktionsenzymen (z.B. durch Spaltung mit EcoRI im Falle von Gen 1 und durch partielle Spaltung im Falle von Gen 2), da keine Schnittstellen für übliche Restriktionsenzyme exakt am Beginn und am Ende des Gens vorliegen. Die Stilbensynthase-Gene bzw. die Gen-Einheiten können auch an ihren Enden solche DNA Sequenzen tragen, welche für ihre Handhabung jeweils angepaßt sind (z.B. "Linker").

Die Stilbensynthase-Gene (bzw. die Gen-Einheiten) können in der Form vorliegen, wie sie im Genom von Pflanzen enthalten sind ("genomische" Form, einschließlich nicht Stilbensynthase kodierender und/oder nicht regulatorisch wirkender Sequenzen (wie Introns) oder in einer Form, welche der cDNA ("copy" DNA) entspricht, die über mRNA mit Hilfe von Reverse-Transkriptase/Polymerase erhältlich ist (und keine Introns mehr enthält). Die Stilbensynthase-Gene können auch in teilweise oder vollständig synthetisierter Form vorliegen. Unter synthetischen Genen werden auch solche verstanden, welche durch das neue Zusammenfügen von Teilen natürlicher Gene entstehen.

In den erfindungsgemäßen Stilbensynthase-Genen (bzw. den Gen-Einheiten) können DNA-Abschnitte durch im wesentlichen gleichwirkende andere DNA-Abschnitte oder DNA's ersetzt sein.

Im vorliegenden Zusammenhang soll unter "fremder" DNA, solche DNA (insbesondere Gene bzw. Gen-Einheiten oder deren Teile) verstanden werden, welche in einem bestimmten prokaryontischen oder eukaryontischen Genom nicht natürlich vorkommt, sondern erst durch Eingriffe durch den Menschen in dieses Genom aufgenommen wird. "Zusätzliche" DNA (insbesondere Gene bzw. Gen-Einheiten oder deren Teile) soll solche DNA sein, welche in dem jeweiligen prokaryontischen oder eukaryontischen Genom zwar natürlich vorkommt, jedoch in zusätzlicher Menge durch Eingriffe durch den Menschen in dieses Genom aufgenommen wird. Die "fremde" DNA oder "zusätzliche" DNA kann je nach Bedarf und Art des vorliegenden Falles in einem oder mehreren Exemplaren eingebaut werden.

Stilbensynthase, welche unter Mitwirkung der erfindungsgemäßen Stilbensynthase-Gene (bzw. der Gen-Einheiten) in Pflanzen oder Pflanzenzellen gebildet wird, bedeutet jedes Enzym, welches die Bildung von solchen pflanzlichen Abwehrstoffen gegen Schädlinge (Phytoalexine) bewirkt, die das Stilbengegerüst aufweisen.

Im vorliegenden Fall ist als Stilben Resveratrol besonders bevorzugt, d.h. als Stilbensynthase wird die Resveratrolsynthase besonders bevorzugt.

Wie bereits erwähnt, sind die erfindungsgemäßen Stilbensynthase-Gene solche, die aus Wein, vorzugsweise aus (Vitis vinifera und Vitis riparia), bevorzugt aus Vitis vinifera, insbesondere aus Vitis vinifera der Sorte Optima isoliert werden können.

Besonders bevorzugt werden als erfindungsgemäße Stilbensynthase-Gene die Stilbensynthase-Gene, welche (als Gen-Einheiten) auf den Plasmiden pVSt1, pVSt2 und pVSt12t3 (welche weiter unten näher beschrieben werden) vorliegen, sowie die im wesentlichen gleichwirkenden DNA-Sequenzen.

Die in Weinreben vorkommenden Stilbensynthase-Gene (angenommen wird eine Zahl von wenigstens 5 Genen) weisen über weite Bereiche eine DNA-Sequenzhomologie auf. Die Stilbensynthase-Gene, die auf dem Plasmid pVSt12t3 enthalten sind, liegen im Genom der Pflanzen nahe beieinander (genetisch gekoppelt). Die Stilbensynthase-Gene codieren alle für Resveratrolsynthase, wobei sich die Gene im wesentlichen durch die Quantitäten der gebildeten Resveratrolsynthase unterscheiden. Die im vorliegenden Text nicht detailliert beschriebenen Stilbensynthase-Gene können auf Grund der Sequenzhomologie in üblicher Weise mit den bekannten Methoden der Molekularbiologie ermittelt, isoliert und aufgeklärt werden, wobei dies wegen der Homologien besonders günstig mit Hilfe der Gen-DNA-Sequenzen möglich ist, die sich auf den Plasmiden pVSt12t3, pVSt1 und pVSt2 befinden.

Erfindungsgemäß besonders bevorzugt werden die Stilbensynthase-Gene bzw. Gen-Einheiten, die auf dem Plasmid pVSt12t3 enthalten sind, sowie die im wesentlichen gleichwirkenden DNA-Sequenzen.

Ein erfindungsgemäßes Gen, im folgenden "Gen 1" genannt, liegt als Gen-Einheit, im folgenden "Gen-Einheit 1" genannt, auf einem etwa 4,9 kb großen EcoRI-Fragment auf dem Plasmid pVSt12t3. Die Gen-Einheit 1 kann auch mit BamHI aus dem Plasmid isoliert werden. HindIII schneidet im Gen. Die Gen-Einheit 1 liegt auch auf dem Plasmid pVSt1 vor und kann mit den genannten Restriktionsenzymen auch aus diesem Plasmid isoliert werden.

Ein weiteres erfindungsgemäßes Gen, im folgenden "Gen 2" genannt, liegt als Gen-Einheit, im folgenden "Gen-Einheit 2" genannt, auf einem etwa 3,4 kb großen Fragment auf dem Plasmid pVSt12t3, welches durch partiellen Abbau mit EcoRI erhalten wird. Asp718 schneidet im Gen.

Ein weiteres erfindungsgemäßes funktionelles Stilbensynthase-Gen wird durch die Fusion der Gen-Einheit 2 an der EcoRI-Stelle bei 3600 bp des Plasmids pVSt12t3 mit der EcoRI-Stelle bei 200 bp des Plasmids pVSt12t3 erhalten. Das Fragment der Gen-Einheit 2 wird hierbei durch die Teilsequenz eines weiteren Gens (Gen 3) ergänzt, welches teilweise auf dem Plasmid pVSt12t3 enthalten ist. Die beiden Teilstücke der Gene bzw. Gen-Einheiten 2 und 3 sind auch auf dem Plasmid pVSt2 enthalten. Das so erhaltene Fusions-Gen stellt ein Beispiel für ein synthetisches Gen dar.

Erfindungsgemäß bevorzugt werden die Gene und Gen-Einheiten 1 und 2 sowie das Fusions-Gen und die entsprechende Gen-Einheit. Besonders bevorzugt werden die Gene und Gen-Einheiten 1 und 2.

Der Escherichia coli Stamm E. coli Fier 1 pVSt1 enthält das Plasmid pVSt1. Der Escherichia coli Stamm E. coli Fier 2 pVSt2 enthält das Plasmid pVSt2. Der Escherichia coli Stamm E. coli Fier pVSt12t3 enthält das Plasmid pVSt12t3. Diese Stämme wurden bei der Deutschen Sammlung von Mikroorganismen (DSM), Mascheroder Weg 1b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt (Hinterlegungsdatum: E. coli Fier 1 pVSt1 und Fier 2 pVSt2: 18. Juni 1990 und E. coli Fier pVSt12t3: 11. Februar 1991).

Der Stamm E. coli Fier 1 pVSt1 erhielt die Hinterlegungsnummer DSM 6002, der Stamm E. coli Fier 2 pVSt2 erhielt die Hinterlegungsnummer DSM 6003 und der Stamm E. coli Fier pVSt12t3 erhielt die Hinterlegungsnummer DSM 6346.

Diese Stämme sowie ihre Mutanten sind ebenfalls Teil der vorliegenden Erfindung. Die in diesen Wirten hinterlegten Plasmide pVSt12t3, pVSt1 und pVSt2 können in üblicher Weise durch die Vermehrung der Stämme und anschließende Isolierung der Plasmide leicht in den benötigten Mengen gewonnen werden.

Funktionell vollständige Gene, wie die erfindungsgemäßen Stilbensynthase-Gene, bestehen aus einem regulatorisch wirkenden Teil (insbesondere Promotor) und dem Strukturgen, welches das Protein Stilbensynthase kodiert.

Beide Genteile können unabhängig voneinander verwendet werden. So ist es möglich, dem regulativ wirkenden Teil eine (vom Stilbensynthase-Gen abweichende) andere DNA-Sequenz nachzuschalten, welche nach dem Einbau in das Pflanzengenom exprimiert werden soll. Da nur wenige isolierte Promotoren bekannt sind, welche ihre Wirkung in Pflanzen bzw. Pflanzenzellen entfalten können, stellen die Promotoren der Stilbensynthase-Gene, welche ebenfalls Bestandteile der vorliegenden Erfindung sind, wertvolle Hilfsmittel bei der Erzeugung transformierter Pflanzen bzw. Pflanzenzellen dar.

Ebenso ist es möglich, den Stilbensynthase-Struktur-Genen einen "fremden" regulatorisch wirkenden Teil vorzuschalten. Dies könnte vorteilhaft sein, wenn bei bestimmten Pflanzen nur bestimmte (z.B. pflanzeneigene) regulatorisch wirkende Gene ausreichend wirksam werden können Die Stilbensynthase-Struktur-Gene stellen somit wertvolle, selbstständig einsetzbare Einheiten dar und sind, wie bereits dargelegt, ebenfalls Teil der vorliegenden Erfindung. Das erfindungsgemäße Stilbensynthase-Gene können nach den üblichen Methoden in die regulatorisch wirkenden Teile und die Struktur-Gene getrennt werden. Es ist auch möglich, Teile von verschiedenen natürlich vorkommenden Stilbensynthase-Genen zu neuen funktionellen "synthetischen" Genen zu kombinieren (z.B. die oben beschriebene Kombination der Gene 2 und 3). Bevorzugt werden die vollständigen natürlichen erfindungsgemäßen Stilbensynthase-Gene (bzw. die Gen-Einheiten) verwendet.

Mit Hilfe der üblichen Methoden ist es möglich, die Stilbensynthase-Gene (bzw. die Gen-Einheiten) oder ihre Teile ein oder mehrfach (z.B. Tandemanordnung), vorzugsweise einfach, in beliebige prokaryontische (vorzugsweise bakterielle) oder eukaryontische (vorzugsweise pflanzliche) DNA als "fremde" oder "zusätzliche" DNA einzubauen. So können z.B. die Gene 1 und 2 (bzw. ihre Gen-Einheiten) gemeinsam in der Anordnung, wie sie auf dem Plasmid pVSt12t3 liegen, eingebaut werden. Die so "modifizierte" rekombinante DNA, welche z.B. zur Transformation von Pflanzen bzw. Pflanzenzellen verwendet werden kann und nach der Transformation in Pflanzen bzw. Pflanzenzellen enthalten ist, ist Bestandteil der vorliegenden Erfindung.

Die Stilbensynthase-Gene (bzw. die Gen-Einheiten) und/oder ihre Teile sowie die rekombinante DNA können als "fremde" oder "zusätzliche" DNA in Vektoren (insbesondere Plasmiden, Cosmiden oder Phagen), in transformierten Mikroorganismen (vorzugsweise Bakterien, insbesondere Gram-negativen Bakterien, wie E. coli) sowie in transformierten Pflanzenzellen und Pflanzen bzw. in deren DNA enthalten sein. Solche Vektoren, transformierte Mikroorganismen (die auch diese Vektoren enthalten können) sowie die transformierten Pflanzenzellen und Pflanzen und deren DNA stellen Bestandteile der vorliegenden Erfindung dar.

Wie bereits angedeutet, werden erfindungsgemäß die Stilbensynthase-Gene (bzw. die Gen-Einheiten) ein- oder mehrfach (an gleichen oder verschiedenen Stellen des Genoms) in das natürliche pflanzliche Genom eingebaut, wobei die verschiedenen Gene auch mit einander kombiniert werden können. Bei Pflanzen, welche bereits über die Fähigkeit der Stilbensynthese verfügen, kann der Einbau eines oder mehrerer erfindungsgemäßer Stilbensynthasegene zu einem erheblich verbesserten Resistenzverhalten führen. Gegebenenfalls werden nur die erfindungsgemäßen Strukturgene verwendet, wobei ein evtl. aus der jeweiligen Pflanze isoliertes regulatorisches DNA Element vorgeschaltet wird.

Die erhöhte Resistenz der erfindungsgemäßen transformierten Pflanzenzellen und Pflanzen ist von Bedeutung für Landwirtschaft und Forsten, für den Zierpflanzenanbau, den Heilpflanzenanbau und die Pflanzenzucht. Auch bei der Kultivierung von Pflanzenzellen, z.B. zur Gewinnung von pharmazeutisch brauchbaren Stoffen, ist es von Vorteil, Pflanzenzellen verfügbar zu haben, welche gegen den Befall durch mikrobielle Schädlinge, insbesondere Pilze, erhöhte Resistenzen aufweisen.

Die vorliegende Erfindung betrifft somit auch ein Verfahren zur Herstellung transformierter Pflanzenzellen (einschließlich Protoplasten) und Pflanzen (einschließlich Pflanzenteile und Samen) mit erhöhter Resistenz gegen Schädlinge, welches dadurch gekennzeichnet ist, daß man

(a) ein oder mehrere Stilbensynthase-Gene (bzw. Gen-Einheiten) aus Wein und/oder Teile der Stilbensynthase-Gene (bzw. der Gen-Einheiten) aus Wein und/oder erfindungsgemäße rekombinante DNA in den Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls vermehrt und gegebenenfalls

(c) von den so erhaltenen transformierten Pflanzen der Elterngeneration oder weiterer daraus gewonnener Generationen die gewünschten Pflanzenteile (einschließlich Samen) gewinnt.

Die Verfahrensschritte (a), (b) und (c) können nach bekannten Verfahren und Methoden in üblicher Weise durchgeführt werden.

Transformierte Pflanzenzellen (einschließlich Protoplasten) und Pflanzen (einschließlich Pflanzenteile und Samen), welche ein oder mehrere Stilbensynthase-Gene (bzw. Gen-Einheiten) aus Wein und/oder Teile der Stilbensynthase-Gene (bzw. der Gen-Einheiten)aus Wein als "fremde" oder "zusätzliche" DNA enthalten sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung der Stilbensynthase-Gene (bzw. der Gen-Einheiten) aus Wein und/oder ihrer Teile und/oder der erfindungsgemäßen rekombinanten DNA und/oder der erfindungsgemäßen rekombinanten Vektoren und/oder der erfindungsgemäßen transformierten Mikroorganismen zur Transformation von Pflanzenzellen (einschließlich Protoplasten) und Pflanzen (einschließlich Pflanzenteilen und Samen) sowie die

(b) Verwendung der erfindungsgemäßen transformierten Pflanzenzellen (einschließlich Protoplasten) und Pflanzen (einschließlich Pflanzenteilen und Samen) zur Erzeugung von Vermehrungsmaterial sowie zur Erzeugung neuer Pflanzen und deren Vermehrungsmaterial und allgemein die

(c) Verwendung der erfindungsgemäßen Stilbensynthase-Gene (bzw. der Gen-Einheiten) aus Wein und/oder ihrer Teile und/oder der erfindungsgemäßen rekombinanten DNA zur Bekämpfung von Schädlingen.

Eine Anzahl verschiedener Methoden steht zur Verfügung, die Stilbensynthase-Gene bzw. die Gen-Einheiten oder ihre Teile als "fremde" oder "zusätzliche" DNA in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Das genetische Material, welches für Stilbensynthase kodiert, wird in die T-DNA von geeigneten Ti-Plasmiden eingesetzt (z.B. Zambryski et al. 1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation von gereinigter DNA, die das gewünschte Gen enthält in Pflanzenprotoplasten (z.B. Hain et al., 1985; Krens et al., 1982; Paszkowski et al., 1984) in Gegenwart von Polykationen oder Calziumsalzen und Polyethylenglykol.

Die DNA-Aufnahme kann auch zusätzlich durch ein elektrisches Feld (Elektroporation) begünstigt werden (z.B. Fromm et al. 1986).

Die DNA kann in bekannter Weise auch über Pflanzenpollen eingeführt werden, indem Pollen mit physikalisch beschleunigten Partikeln "beschossen" werden, welche die DNA tragen (vgl. EP-A 0 270 356).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens (gemäß der Methode aus EP-A 116 718) werden die Gene bzw. Geneinheiten aus den Plasmiden pVSt12t3 oder pVSt1 in einen geeigneten intermediaeren E.coli Vektor z.B. pCV001 oder pCV002, (vergl. EP-A-116 718; Koncz et al. 1986) bzw. vorzugsweise Derivaten davon, die zusätzlich ein Reportergen wie z.B. nptII (Herrera-Estrella et al. 1983) oder hpt (Van den Elzen et al 1986) enthalten, kloniert.

Das so konstruierte Plasmid wird auf Agrobacterium tumefaciens, das z.B. pGV3850 bzw. Derivate davon enthält (Zambryski et al. 1983) mit üblichen Methoden (z.B. Van Haute et al. 1983) übertragen. Es können so auch die Gene 1 und 2 gemeinsam übertragen werden. Alternativ dazu kann die Stilbensynthase-Geneinheit in einem binaeren Vektor (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die Stilbensynthase-Gene bzw. Gen-Einheiten in einer auf Pflanzen transferierbaren Form enthält wird, im weiteren zur Pflanzentransformation verwendet.

In einer weiteren bevorzugten Ausführungsform wird das Plasmid pVSt12t3 oder das isolierte Plasmid pVSt1 oder die isolierten Gene bzw. Gen-Einheiten gegebenenfalls zusammen mit einem anderen Plasmid, das ein Reportergen für Pflanzenzellen, z.B. für Kanamycin-Resistenz (z.B. Herrera-Estrella et al. 1983) oder eine Hygromycin-Resistenz (van den Elzen, 1986) enthält, vorzugsweise pLGVneo2103 (Hain et al. 1985), pLGV23neo (Herrera-Estrella 1983), pMON129 (Fraley R.T. et al., Proc. National Acad. Sci. USA 80, 4803 (1983), pAK1003, pAK 2004 (Velten J. et al., EMBO Journ. Vol. 3, 2723 (1984) oder pGSSTneo3 (pGSST3) (EP-A-189 707), in üblicher Weise durch direkten Gentransfer auf Pflanzenprotoplasten übertragen (z.B. Hain et al 1985). Dabei können das bzw. die Plasmide oder die Gene bzw. die Gen-Einheiten in zirkulärer, vorzugsweise jedoch in linearer Form, vorliegen. Bei der Verwendung eines Plasmids mit Reportergen werden Kanamycin-resistente Protoplasten dann auf Expression von Stilbensynthase überprüft. Im anderen Fall (ohne Reportergen) werden die resultierenden Kalli auf die Expression des oder der Stilbensynthase-Gene geprüft (Screening mit üblichen Methoden).

Transformierte (transgene) Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycinsulfat in vitro (Reiss et al. 1984; Schreier et al. 1985) oder durch die Expression der Nopalinsynthase (nach Aerts et al. 1983) oder Stilbensynthase durch Northern-Blot-Analyse und Western Blot-Analyse nachgewiesen. Die Stilbensynthase und die Stilbene können auch in bekannter Weise mit Hilfe spezifischer Antikörper in transformierten Pflanzen nachgewiesen werden. Stilbensynthase kann auch durch Enzymaktivitätstest nachgewiesen werden (Rolfs et al., Plant Cell Reports 1, 83-85, 1981).

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche die erfindungsgemäßen Stilbensynthase-Gene (bzw. die Gen-Einheiten) enthalten und welche Bestandteile der vorliegenden Erfindung sind, zeigen eine erheblich höhere Resistenz gegen Schädlinge, insbesondere pflanzenpathogene Pilze.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen und Pflanzenzellen, welche zur Vermehrung der transformierten Pflanzen und Pflanzenzellen verwendet werden können und ist somit ebenfalls Teil der vorliegenden Erfindung.

Im vorliegenden Zusammenhang bedeutet der Ausdruck "im wesentlichen gleichwirkende DNA-Sequenzen", daß die Erfindung auch solche Modifikationen umfaßt, bei welchen die Funktion der Stilbensynthase-Gene und ihrer Teile nicht derart beeinträchtigt ist, daß Stilbensynthase nicht mehr gebildet wird oder der regulatorische Genteil nicht mehr wirksam wird. Entsprechende Modifikationen können durch den Ersatz, die Hinzufügung und/oder die Entfernung von DNA-Abschnitten, einzelner Kodons und/oder einzelner Nucleotide erfolgen.

Bei den erfindungsgemäß verwendbaren Mikroorganismen bedeutet "Mutanten" solche modifizierten Mikroorganismen, welche noch die für die Ausführung der Erfindung wesentlichen Merkmale aufweisen, insbesondere die jeweiligen Plasmide enthalten.

Zu den Pflanzen, welchen durch den Einbau (Transformation) der erfindungsgemäßen Stilbensynthase-Gene (bzw. der Gen-Einheiten) Resistenz bzw. eine erhöhte Resistenz gegenüber den obigen Schädlingen verliehen werden kann, gehören praktisch alle Pflanzen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernden Pflanzen, z.B. Getreide (insbesondere Weizen, Roggen, Gerste, Hafer, Hirse, Reis und Mais), Kartoffel, Leguminosen (wie Hülsenfrüchte und insbesondere Alfalfa, Sojabohnen), Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen, wie Rauwolfia und Digitalis. Besonders bevorzugt seien Kartoffel, Tomaten und Leguminosen genannt. Vorzugsweise werden die erfindungsgemäßen Stilbensynthase-Gene als "fremde" DNA in den Genom von Pflanzen eingebaut (also in Pflanzen, ausgenommen Wein).

Als Schädlinge, gegen welche mit Hilfe der erfindungsgemäßen Stilbensynthase-Gene Resistenzen, bzw. erhöhte Resistenzen erzielt werden können, seien tierische Schädlinge, wie Insekten, Milben und Nematoden sowie mikrobielle Schädlinge, wie phytopathogene Pilze, Bakterien und Viren genannt. Besonders hervorgehoben werden mikrobielle Schädlinge, insbesondere phytopathogene Pilze.

Zu den schädlichen Insekten gehören insbesondere Insekten der Ordnungen:

Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere:

Tarsonemus spp., Panonychus spp. und Tetranychus spp.

Zu den schädlichen Nematoden gehören insbesondere:

Pratylenchus spp., Heterodera spp. und Meloidogyne spp.

Zu den mikrobiellen Schädlingen gehören insbesondere die phytopathogenen Pilze:

Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Zu den Viruserkankungen gehören insbesondere Mosaik-, Verzwergungs- und Vergilbungsvirosen.

Beispielhaft aber nicht begrenzend seien einige Erreger von virösen, pilzlichen und bakteriellen Erkrankungen, die unter die oben aufgezählten Oberbegriffe fallen, genannt:

Barley Yellow Dwarf Virus (BYDV), Potato Virus Y (PVY), Cucumber Mosaic Virus (CMV), Watermelon Mosaic Virus (WMV), Tristeza-Virus, Tobacco Mosaic Virus (TMV), Tobacco Necrosis Virus (TNV), Beet necrotic Yellow Vein Virus (BNYVV), Rhizomania-Virus.

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielwseise Pseudocerco sporella herpotrichoides. Weiterhin sei Helminthosporium carbonum aufgeführt.

Die vorliegende Erfindung soll anhand der folgenden beispielhaften Ausführungen näher erläutert werden:

1. Isolierung des Gens für Stilbensynthase aus Wein

Pflanzen und Zellkulturen aus Wein (Vitis vinifera, Sorte Optima) enthalten die Gene für Stilbensynthase, welche die Bildung von Resveratrol Synthase (Größe des Proteins 45 000 D; Reaktion mit spezifischem Antiserum) bewirken.

Bei der Isolierung der Stilbensynthase-Gene wurden die bekannten Verfahren und Methoden der Molekularbiologie verwendet, wie sie beispielsweise in folgendem Handbuch detailliert beschrieben werden: Maniatis, T., Fritsch, E.F., Sambrook, J.: Molecular Cloning: A Laboratory Manual; Cold Spring Harbor Laboratory, Second Edition 1989.

Es wird zunächst eine "Gen-Bibliothek" für Wein angelegt: Genomische DNA aus angereicherten Zellkernen (Bedbrook, J., Plant Molecular Biology Newsletter 2, 24, 1981) wird mit dem Restriktions-Enzym NdeII so geschnitten, daß DNA-Fragmente mit einer Durchschnittslänge von etwa 12 000 Nukleotidpaaren entstehen. Diese Fragmente werden in die BamHI-Stelle von Lambda-Phage EMBL4 kloniert (Frischauf et al., J. Mol. Biol. 170, 827-842, 1983), und die Phagen werden in E. coli vermehrt. Die Gesamtheit der Phagen-Population enthält, kloniert in Teilstücken, die gesamte genomische DNA der Weinzellen, und damit auch die Gene für Stilbensynthasen (Multigenfamilie).

Die Gene für Stilbensynthase, ihre mRNA und die Stilbensynthasen-cDNA enthalten jeweils gleiche Nucleinsäuresequenzen, da sie voneinander abgeleitet weden können (Gen→mRNA→cDNA). Dies bedeutet, daß die Gene für Stilbensynthase durch spezifische Hybridisierung mit Stilbensynthase-cDNA (Schröder et al 1988; Melchior und Kindl 1990, EP-A-309 862) bzw. mit spezifischen Oligonukleotiden, die aus den in EP-A-309 862 angegebenen DNA-Sequenzen ableitbar sind, identifizierbar sind. Die Phagen mit den Genen werden durch Hybridisierung identifiziert, dann isoliert und vermehrt. Die in diesem Phagen klonierte genomische DNA aus Wein wird weiter durch Analyse mit verschiedenen Restriktionsenzymen kartiert, und die Position der Stilbensynthase-Gene wird durch weitere Hybridisierungs-Experimente mit cDNA-Sequenzen bzw. synthetischen Oligonukleotiden festgelegt. Schließlich werden die Gen-Einheiten durch Verdau mit EcoRI bzw. durch partiellen Verdau mit EcoRI aus dem Phagen herausgeschnitten, im entsprechend geschnittenen Plasmid-Vektor pUC18 kloniert (Fa. Gibco-BRL GmbH, Eggenstein, Bundesrepublik Deutschland), und als rekombinante Plasmide vermehrt.

Zur Isolierung weiterer erfindungsgemäßer Stilbensynthase-Gene können auf Grund der Sequenzhomologien DNA-Sequenzen als Sonden verwendet werden, welche in den auf den Plasmiden pVSt12t3, pVSt1 und pVSt2 befindlichen Genen enthalten sind. Beispielsweise können auch Oligonucleotide der folgenden Sequenzen verwendet werden, wobei die Ziffern die Stellung der Nukleotide in Bezug zu A der ATG-Sequenz, die am Beginn der codierenden Region steht, angeben:

```
(a)   106   TATGC   TGATT   ACTAT   TTCAG
            AGTCA   CTAAG   AGCGA   GCACA T 146


(b)   147   GACTG   AGTTG   AAGAA   GAAGT
            TCAAT   CGCAT   ATGTA   A 183


(c)   1000  TATGG   TAACA   TGTCT   AGTGC
            ATGTG   TCTTG   TTTAT   TTTGG
            ATGAG   ATGAG   AAAGA   A 1056
```

Diese Sequenzen oder stark homologe Sequenzen sind in den erfindungsgemäßen Stilbensynthase-Genen enthalten, so daß diese auch durch einen Gehalt an diesen Sequenzen oder durch einen Gehalt an zu diesen Sequenzen stark homologen Sequenzen gekennzeichnet werden können.

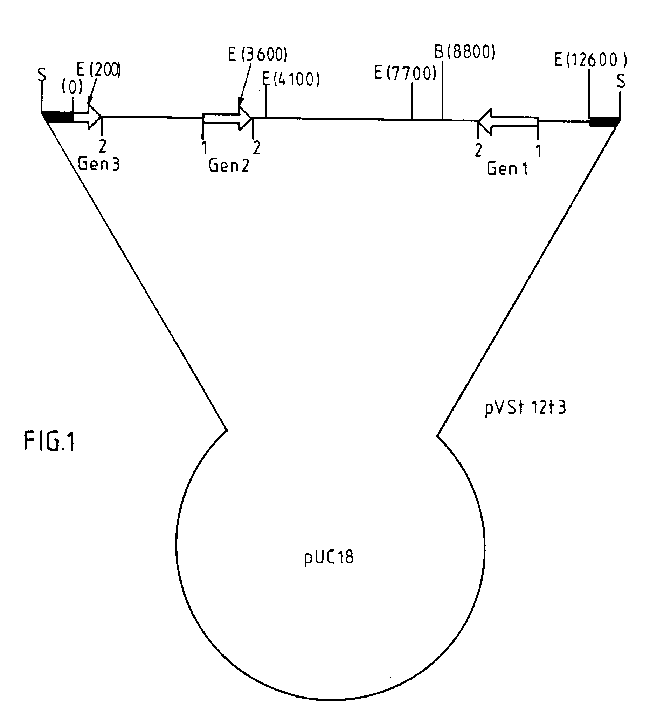

2. Beschreibung der Plasmide pVSt12t3, pVSt1 und pVSt2 (vgl. Fig. 1 - Fig. 3)

A) Die Plasmide pVSt12t3, pVSt1 und pVSt2 bestehen aus zwei Komponenten:

1. Gen-Einheiten bzw. Gen-Teile:

(a) Plasmid pVSt1:

Die Gen-Einheit 1, enthaltend das Struktur-Gen und den Regulator-Anteil des Gens 1 (aus Wein, Sorte Optima) befindet sich auf einer Nucleinsäure, die als DNA-Fragment von (ca) 4900 bp durch Spaltung mit dem Restriktionsenzym EcoRI aus dem Plasmid pVSt1 herausgeschnitten werden kann.

(b) Plasmid pVSt2:

Die Sequenzen von Gen 2 und Gen 3 liegen auf einem 3,4 kb großen EcoRI-Fragment. Nach Zirkularisation dieses Fragmentes entsteht ein funktionelles synthetisches Stilbensynthase-Gen aus den Genteilen der Gene 2 und 3.

(c) Plasmid pVSt12t3:

Das Plasmid pVSt12t3 enthält ein genomisches Fragment von Vitis vinifera von ca. 12600 bp flankiert von γ Phagen DNA im Vektor PUC18. Auf diesem ca. 12600 bp Fragment liegen die kompletten Geneinheiten 1 und 2. Gen 3 liegt nicht komplett auf diesem Fragment. Die Geneinheit 1 kann durch EcoRI Verdau als ein 4900 bp langes Fragment isoliert werden.
Die Geneinheit 2 kann durch partiellen EcoRI Verdau auf einem 3400 bp Fragment isoliert werden.

2. Vektor-Plasmid:

Die Gen-Einheiten bzw. Genteile sind in Vektor pUC18 kloniert. Die Größe des Vektors ist 2686 Nukleotidpaare. Er trägt das Gen für Ampicillin-Resistenz, d.h. E. coli-Zellen mit diesem Plasmid wachsen in Nährmedien, die das Antibiotikum Ampicillin enthalten. Ori: Bezeichnung für Sequenzen, die für die Vermehrung des Plasmids in E. coli notwendig sind.
Die Plasmide pVSt1 bzw. pVSt2 tragen ein Gen für Ampicillin-Resistenz, Sie können in E. coli Zellen, welche pVSt1 bzw. pVSt2 enthalten (E. coli Fier 1 bzw. Fier 2), in üblicher Weise vermehrt werden. Entsprechendes gilt für den E. coli Stamm Fier pVst12t3.
Bevorzugtes Nährmedium für E. coli-Zellen (z.B. JA221, Nakamura, K., Inouye, M., EMBO J. 1, 771-775, 1982) welche pVSt1, pVSt2 und pVSt12t3 enthalten (E. coli Nurdug 2010):

| Bacto-Pepton[*] | 10 g |
|---|---|
| Hefeextrakt | 5 g |
| NaCl | 5 g |
| Agar | 20 g |
| $H_2O$ | 1 l |
| pH 7,5 | |
| Dem Nährmedium werden 50 µg/ml Ampicillin zugefügt. Fermentation: 37°C, aerob | |

(* Bacto ist ein Warenzeichen der Fa. DIFCO Lab. Detroit, USA).

### 3. Transformation von Tabak

a) Kultur von Tabaksprossen und Isolierung von Tabakprotoplasten:

Nicotiana tabacum (Petit Havanna SR1) wird als sterile Sproßkultur auf hormonfreiem LS Medium (Linsmaier und Skoog 1965) vermehrt. In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26°C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 m Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf 1-2 x $10^5$/ml verdünnt. Die Protoplastenkonzentration wird in einer Zählkammer bestimmt.

b) <u>Transformation von regenerierenden Tabakprotoplasten durch Kokultur mit Agrobacterium tumefaciens:</u>

Es wird im folgenden die Methode von Marton et al. 1979 mit kleinen Veränderungen benutzt. Die Protoplasten werden wie beschrieben isoliert und in einer Dichte von 1-2 x $10^5$/ml in K3 Medium (0,4 m Saccharose, 0,1 mg/l NAA, 0,2 ml in K3 Medium (0,4 m Saccharose, 0,1 mg/l NAA, 0,2 mg Kinetin) 2 Tage im Dunkeln und ein bis zwei Tage lang unter Schwachlicht (500 lux) bei 26°C inkubiert, Sobald die ersten Teilungen der Protoplasten auftreten, werden 30 µl einer Agrobakteriumsuspension in minimal A (Am) Medium (Dichte ca. $10^9$ Agrobakterien/ml) zu 3 ml regenerierenden Protoplasten gegeben. Die Kokulturdauer beträgt 3-4 Tage bei 20°C im Dunkeln, Danach werden die Tabakzellen in 12 ml Zentrifugenröhrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von 5 x $10^4$/ml in K3 Medium (0,3 m Saccharose) mit 1 mg/l NAA (Naphthyl-1-essigsäure), 0,2 mg/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert. Die Selektion mit Kanamycin (100 mg/l Kanamycinsulfat (Sigma), 660 mg/g aktives Km) wird 2-3 Wochen nach der Kokultur in Agarose "bead type culture" (Shillito et al. 1983) gestartet, Kanamycinresistente Kolonien können 3-4 Wochen nach Beginn der Selektion vom Hintergrund zurückgebliebener Kolonien unterschieden werden.

c) Direkte Transformation von Tabakprotoplasten mit DNA. Calciumnitrat-PEG Transformation.

In einer Petrischale werden ca. $10^6$ Protoplasten in 180 μl K3 Medium mit 20 μl wäßriger DNA Lösung welche 0,5 μg/μl Plasmid pVSt12t3 oder z.B. 0,5 μg/μl Plasmid pVSt1 gemischt mit 0,5 μg/μl pLGV neo 2103 (Hain et al. 1985) enthält, vorsichtig gemischt. Anschließend werden 200 μl Fusionslösung (0,1 m Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer kleinen Menge K3 Medium aufgenommen und wie im nächsten Abschnitt beschrieben kultiviert. Alternativ können die Protoplasten nach Hain et al. 1985 tranformiert werden.

Die Transformation kann auch ohne den Zusatz der 0,5 μg/μl pLGVneo2103 durchgeführt werden. Da in diesem Fall kein Reportergen eingesetzt wird, werden die resultierenden Kalli auf das Vorhandensein der Stilbensynthase-Gen-Einheit mit Hilfe einer Dot-Blot-Hybridisierung überprüft. Als Hybridisierungs-Probe sind interne EcoRI-Fragmente aus pVSt1 bzw. pVSt2 verwendbar. Selbstverständlich können auch andere Nachweismethoden, wie Test mit Antikörpern oder Feststellung einer erhöhten Pilz-Resistenz eingesetzt werden.

d) <u>Kultur der mit DNA inkubierten Protoplasten und Selektion Kanamycin resistenter Kalli:</u>

Für die im folgenden beschriebene Kultur und Selektion Kanamycin resistenter Kolonien wird eine modifizierte "Bead Type culture"-Technik (Shillito et al, 1983) verwendet. Eine Woche nach Behandlung der Protoplasten mit DNA (vgl. c) werden 3 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoklaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt.

Pro Woche wird das Austauschmedium (K3 + Km) um 0,05 m an Saccharose (ca. 60 mOsm) reduziert.

Schema der Selektion kanamycinresistenter Tabakkolonien nach DNA Transformation:

|   | 0,4 M | 0,3 M | 0,25 M | 0,20 M | 0,15M | 0,10 M | Saccharose im Flüssigmedium |
|---|---|---|---|---|---|---|---|
| A | E S |  |  |  | K |  |  |
|   | 1 | 2 | 3 | 4 | 5 | 6 | Wochen nach DNA Aufnahme |
| (K3 Medium 1 mg NAA, 0,2 mg Kinetin) A = DNA Aufnahme E = Einbettung in Agarose S = Selektion mit Kanamycin (100 mg/l Kanamycinsulfat) K = Kanamycinresistente Kolonien können vom Hintergrund eindeutig unterschieden werden | | | | | | | |

e) <u>Regeneration kanamycinresistenter Pflanzen:</u>

Sobald die kanamycinresistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24°C im Kulturraum gehalten. Die andere Hälfte wird als Kalluskultur auf LS Medium mit 1 mg/l NAA, 0,2 mg/l Kinetin, 0,1 mg/1 BAP und 100 mg/l Kanamycinsulfat propagiert. Wenn die regenerierten Sproße ca. 1 cm groß sind, werden sie abgeschnitten und auf 1/2 LS Medium (1 % Saccharose, 0,8 % Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

f) <u>Transformation von Blattscheiben durch Agrobacterium tumefaciens</u>

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechender Agrobacterien

(ca. $10^9$/ml) (vgl. b) in Am-Medium, siehen unten) für ca. 5 Minuten inkubiert. Die infizierten Blattstücke werden auf MS-Medium (siehe unten) ohne Hormone für 3-4 Tage bei ca. 24°C gehalten. Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 µg/ml Cefotaxim und 100 µg/ml Kanamycinsulfat (Sigma) gelegt. Nach zwei Wochen sollte das Medium erneuert werden. Transformierte Sproße werden nach weiteren 2-3 Wochen sichtbar. Die Regeneration von Sproßen sollte parallel auch ohne Selektionsdruck durchgeführt werden. Die regenerierten Sproße müssen dann durch biologische Tests z.B. auf Nopalinsynthase oder Stilbensynthase Aktivität auf Transformation getestet weren. Auf diese Weise werden 1-10 % transformierte Sproße erhalten.

Biochemische Nachweismethode der Transformation

Nachweis von Nopalin in Pflanzengeweben:

Nopalin wird wie bei Otten und Schilperoort (1978) und Aerts et al. (1979) beschrieben, wie folgt, nachgewiesen. 50 mg Pflanzenmaterial (Kallus oder Blattstücke) werden über Nacht in LS Medium mit 0,1 M Arginin bei Raumtemperatur in einem Eppendorfgefäß inkubiert. Das Pflanzenmaterial wird danach auf saugfähigem Papier abgetupft, in einem frischen Eppendorfzentrifugengefäß mit einem Glasstab homogenisiert und 2 Min. in einer Eppendorfzentrifuge zentrifugiert. 2 µl des Überstandes werden auf ein für Elektrophorese geeignetes Papier (Whatman 3 MM Papier) (20 x 40 cm) punktförmig aufgetragen und getrocknet. Das Papier wird mit dem Laufmittel (5 % Ameisensäure, 15 % Essigsäure, 80 % $H_2O$, pH 1,8) getränkt und bei 400 V für 45 Minuten elektrophoretisiert. Nopalin läuft zur Kathode hin. Das Papier wird dann mit einem Luftstrom heiß getrocknet und durch Phenanthrenchinon-Färbemittel (gleiches Volumen 0,02 % Phenanthrenchinon in Ethanol und 10 % NaOH in 60 % Ethanol) in Laufrichtung gezogen. Das getrocknete Papier wird unter langwelligem UV-Licht betrachtet und fotografiert. Arginin und Argininderivate werden mit dem Reagenz gelb fluoreszierend gefärbt.

Neomycin-Phosphotransferase (NPT II) Enzymtest:

NPT II Aktivität in Pflanzengewebe wird durch in situ Phosphorylierung von Kanamycin, wie bei Reiß et al. (1984) beschrieben und von Schreier et al. (1985) modifiziert, wie folgt, nachgewiesen. 50 mg Pflanzengewebe werden in 50 µl Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 µl des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0,165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min. lang und einmal 30 Min. mit Reaktionspuffer gewaschen (67 mM Tris-Maleat, pH 7,1, 42 mM $MgCl_2$, 400 mM Ammoniumchlorid). Das Gel wird auf eine gleichgroße Glasplatte gelegt und mit 40 ml 1 %iger Agarose in Reaktionspuffer, der die Substrate Kanamycinsulfat (20 µg/ml) und 20-200 µCi $^{32}$P ATP (Amersham) enthält, überschichtet. Das Sandwichgel wird 30 Min. bei Zimmertempreratur inkubiert und dann wird ein Blatt Phosphozellulosepapier P81 (Whatman) auf die Agarose gelegt. Darüber werden vier Filtrierpapierlagen 3 MM, (Whatman) und einige Papierhandtücher gestapelt. Der Transfer von in situ phosphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7.5 bei 80°C gewaschen, getrocknet und für 1-12 h lang bei -70°C autoradiografiert (XAR5 Film Kodak).

4. Transformation von Solanum tuberosum (Kartoffel)

Die Transformation wurde genau nach dem in der EP-A-0 242 246, Seiten 14 bis 15 angegebenen Weise transformiert, wobei die Agrobakterien Ti-Plasmide enthalten, die das Stilbensynthese-Gen oder die Stilbensynthase-Gene tragen.

Alle Prozentangaben in den obigen Beispielen beziehen sich auf Gewichtsprozente, wo nichts anderes angegeben wird.

In den gemäß den obigen Beispielen erhaltenen Pflanzenzellen und Pflanzen (Tabak) wurde die Anwesenheit der Stilbensynthase-Gene durch Southern Blot Analyse bestätigt. Die Expression der Stilbensynthase-Gene wurde durch Northern Blot Analyse, Stilbensynthase und Stilbene mit Hilfe von spezifischen Antikörpern nachgewiesen. Transformierte und nicht-transformierte Pflanzen (zum Vergleich) wurden mit einer Sporensuspension von Botrytis cinera besprüht und nach 1 Woche der Pilzbefall bonitiert. Die transformierten Pflanzen zeigten (gegenüber den nicht transformierten Vergleichspflanzen) eine erhöhte Resistenz gegen Pilzbefall.

Im folgenden werden einige der bei der Transformation von Pflanzen bzw. Pflanzenzellen eingesetzte Medien beschrieben:

Am-Medium

| | |
|---|---|
| 3,5 g | $K_2HPO_4$ |
| 1,5 g | $KH_2PO_4$ |
| 0,5 g | $Na_3$ Citrat |
| 0,1 g | $MgSO_4$ x $7H_2O$ |
| 1 g | $(NH_4)_2SO_4$ |
| 2 g | Glukose |
| | ad 1 l |

Medium für sterile Soroßkultur von Tabak

| Macro-elemente | 1/2 der Konzentration der MS Salze | |
|---|---|---|
| Micro-elemente | 1/2 der Konzentration der MS Salze | |
| Fe-EDTA | Murashige und Skoog (MS) | |
| Myo-Inosit | | 100 mg/l |
| Sucrose | | 30 g/l |
| Agar | | 8 g/l |
| Vitamine | Ca-panthotenat | 1 mg/l |
| | Biotin | 10 mg/l |
| | Nicotinsäure | 1 mg/l |
| | Pyridoxin | 1 mg/l |
| | Thiamin | 1 mg/l |
| pH 5,7 vor dem Autoklavieren | | |

K3-Medium

Zur Kultur von Nicotiana tabacum petit Havana SR1, Nicotiana tabacum Wisconsin 38, und Nicotiana plumaginifolia Protoplasten (Nagy und Maliga, 1976)

| Macro-elemente | $NH_4NO_3$ | 250 mg/l |
|---|---|---|
| | $KNO_3$ | 2500 mg/l |
| | $CaCl_2 \cdot 2H_2O$ | 900 mg/l |
| | $MgSO_4.7H_2O$ | 250 mg/l |
| | $NaH_2PO_4.1H_2O$ | 150 mg/l |
| | $(NH_4)_2SO_4$ | 134 mg/l |
| | $CaHPO_4.1H_2O$ | 50 mg/l |

(fortgesetzt)

| Micro-elemente | $H_3BO_3$ | 3 mg/l |
|---|---|---|
| | $MnSO_4.1H_2O$ | 10 mg/l |
| | $ZnSO_4.4H_2O$ | 2 mg/l |
| | KI | 0,75 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Sucrose | | 137 g/l |
| | | (= 0,4 M) |
| Xylose | | 250 mg/l |
| Vitamine | Nicotinsäure | 1 mg/l |
| | Pyridoxin | 1 mg/l |
| | Thiamin | 10 mg/l |
| Hormone | NAA | 1,0 mg/l |
| | Kinetin | 0,2 mg/l |
| pH 5,6 Filter sterilisieren | | |

<u>Linsmaier und Skoog Medium</u> (Linsmaier und Skoog 1965)

Zur Kultur von regenerierenden Protoplasten und für Gewebekultur von Tabaktumoren und Kallus. Linsmaier und Skoog (LS) Medium ist Murashige und Skoog Medium (Murashige und Skoog, 1962) mit den folgenden Modifikationen:

- Thiamin wird in höherer Konzentration eingewogen 0,4 mg/l anstatt 0,1 mg/l;
- Glycin, Pyridoxin und Nicotinsäure fehlen.

| Macro-elemente | $NH_4NO_3$ | 1650 mg/l |
|---|---|---|
| | $KNO_3$ | 1900 mg/l |
| | $CaCl_2.2H_2O$ | 440 mg/l |
| | $MgSO_4.7H_2O$ | 370 mg/l |
| | $KH_2PO_4$ | 170 mg/l |

(fortgesetzt)

| Micro-elemente | $H_3BO_3$ | 6,2 mg/l |
| | $MnSO_4.1H_2O$ | 22,3 mg/l |
| | $ZnSO_4.4H_2O$ | 8,6 mg/l |
| | KI | 0,83 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Saccharose | | 30 g/l |
| Agar | | 8 g/l |
| Vitamine | Thiamin | 0,4 mg/l |
| Hormone: | NAA | 1 mg/l |
| | Kinetin | 0,2 mg/l |
| pH 5,7 vor dem Autoklavieren | | |

Zur Transformation von Pflanzen bzw. Pflanzenzellen kann die folgende Literatur angeführt weren:

Aerts M, Jacobs M, Hernalsteens JP, Van Montagu M, Schell J (1983) Induction and in vitro culture of Arabidopsis thaliana crown gell tumours. Plant Sci Lett. 17: 43-50

Fraley R.T., Rogers S.G., Horsch R.B., Sanders P.R., Flick J.S., Adams S.P., Bittner M.L., Brand L.A., Fink C,L., Fry J.S., Fallupi G.R., Goldberg S.B., Hoffmann N,L., Woo S.C. (1983). Expression of bacterial genes in plant cells. Proc. Natl. Acad. Sci. USA 80:4803-4807.

Fromm ME, Taylor LP, Walbot V (1986) Stable transformation of maize after gene transfer by electroporation. Nature 319: 791-793

Hain, R., Stabel, P., Czernilofsky, A.P., Steinbiß, H.H., Herrera-Estrella, L., Schell, J. (1985) Uptake, integration, expression and genetic transmission of a selectable chimeric gene by plant protoplasts. Molec Gen Genet 199: 161-168

Hain R., Bieseler B., Kindl H., Schröder G., Stöcker R. (1990) Expression of a stilbene synthase gene in Nicotiana tabacum results in synthesis of the phytoalexin resveratrol. Plant Mol, Biol, 15:325-336.

Hernalsteens JP, Thia-Tong L, Schell J, Van Montagu M (1984) An Agrobacterium-transformed Cell culture from the monocot Asparagus officinalis, EMBO J 3:3039-3041

Herrera-Estrella L., De Block M., Messens E., Hernalsteens JP., van Montagu M., Schell J, (1983) EMBO J. 2: 987-995.

Horsch RB, Fry JE, Hoffmann NL, Eichholtz D, Rogers SG, Fraley RT (1985) A simple and general method for transferring genes into plants. Science 277: 1229-1231

Krens FH, Molendijk L, Wullems GJ, Schilperoort RA (1982) in vitro transformation of plant protoplasts with Ti-plasmid DNA. Nature 296: 72-74

Koncz C, Schell J (1986) The promotor of $T_L$-DNA gene 5 controls the tissue-specific expression of chimaeric genes carried by a noval type of Agrobacterium linary vector. Mol. Gen. Genet. (1986) 204: 338-396

Linsmaier DM, Skoog F (1965) Organic growth factor requirements of tobacco tissue cultures. Physiol Plant 18: 100-127

Marton L, Wullems GJ, Molendijk L, Schilperoort PR (1979) In vitro transformation of cultured cells from Nicotiana tabacum by Agrobacterium tumefaciens. Nature 277: 1229-131

Melchior F, Kindl H (1990) Grapevine stilbene synthase cDNA only slightly differing from chalcone synthase cDNA is expressed in Escherichia coli into a catalytically active enzyme FEBS 268:17-20

Nagy JI, Maliga P (1976) Callus induction and plant regeneration from mesophyll protoplasts of Nicotiana sylvestris. Z Pflanzenphysiol 78: 453-455

Otten LABM, Schilperoort RA (1978) A rapid microscale method for the detection of Lysopin and Nopalin dehydrogenase activities. Biochim biophys acta 527: 497-500

Paszkowski J, Shillito RD, Saul M, Mandak V, Hohn T, Hohn B, Potrykus I (1984) Direct gene transfer to plants. EMBO J 3: 2717-2722

Rolf, C.H., Fritzemeier K.H. and Kindl H. (1981) Cultured cells of Arachis hypogaea susceptible to induction of stilbene synthase (resveratrol forming) Plant Cell. Rep. 1:83-85

Schröder, G., Brown J.W.S. and Schröder, J. (1988) Molecular analysis of resveratrol synthase: cDNA, genomic clones and relationship with chalconsynthase. Eur. J.Biochem. 172, 161-169

Shillito RD, Paszkowski J. Potrykus I (1983) Agarose plating and Bead type culture technique enable and stimulate development of protoplast-derived colonies in an number of plant species. Pl Cell Rep 2: 244-247 Van den Elzen PJM, Townsend J, Lee KY, Bedbrook JR (1985) A chimaeric resistance gen as a selectable marker in plant cells. Plant Mol. Biol, 5, 299-302.

Van den Elzen PJM, Townsend J, Lee KY, Bedbrook JR (1985) A chimaeric resistance gen as a selectable marker in plant cells. Plant Mol. Biol. 5, 299-302.

Velten J, Velten L, Hain R, Schell J (1984) Isolation of a dual plant promotor fragment from the Ti Plasmid of Agrobacterium tumefaciens. EMBO J 12: 2723-2730

Van Haute E, Joos H, Maes M, Warren G, Van Montagu M, Schell J (1983) Intergenic transfer and exchatge recombination of restriction fragments clones in pBR 322: a novel strategy for the reversed genetics of Ti plasmids of /Agrobacterium tumefacines, EMBO J 2: 411-418.

Zambryski P, Joos H, Genetello C, van Montagu M, Schell J (1983) Ti-plasmid vector for the introduction of DNA into plant cells without altering their normal regeneration capacity, EMBO J 12: 2143-2150,

Reiss, B., Sprengel, Will H., and Schaller H(1984) A new sensitive method for qualitative and quantitative assay of neomycin phosphotransferase in crude cell tracts, GENE 1081: 211-217

Schreier P.H., Seftor E.A., Schell J. and Bohnert H.J. (1985) The use of nuclear-encoded sequences to direct the light-regulated synthesis and transport of a foreingn protein into plant chloroplasts, EMBO J Vol. 4, No. 1: 25-32

Weiterhin können die folgenden veröffentlichten Patentanmeldungen aufgeführt werden:

| EP-A 116 718 | EP-A-126 546 |
|---|---|
| EP-A 159 418 | EP-A-164 597 |
| EP-A 120 515 | EP-A-175 966 |
| EP-A-120 516 | WO 84/02913 |
| EP-A-172 112 | WO 84/02919 |
| EP-A-140 556 | WO 84/02920 |
| EP-A-174 166 | WO 83/01176 |
| EP-A-122 791 | |

Erläuterungen zu Fig. 1 - Fig. 4

Fig. 1    stellt das Plasmid pVSt12t3 dar. Die Stilbensynthase-Geneinheiten 1 und 2 liegan auf einem ca. 12.600 bp langen Vitis DNA Fragment, das in pVSt12t3 von λ Phagen DNA flankiert, in die SmaI Stelle von pUC18 kloniert wurde. Von Gen 3 liegen nur terminale Sequenzen auf pVSt12t3. Mit 1 ist jeweils der Anfang der kodierenden Region und mit 2 das Ende der kodierenden Region von Gen 1, Gen 2 und Gen 3 angegeben. Die Pfeile geben zudem die Orientierung der Gene auf dem Fragment an, Die fett gezeichneten Bereiche repräsentieren flankierende Phagen DNA.

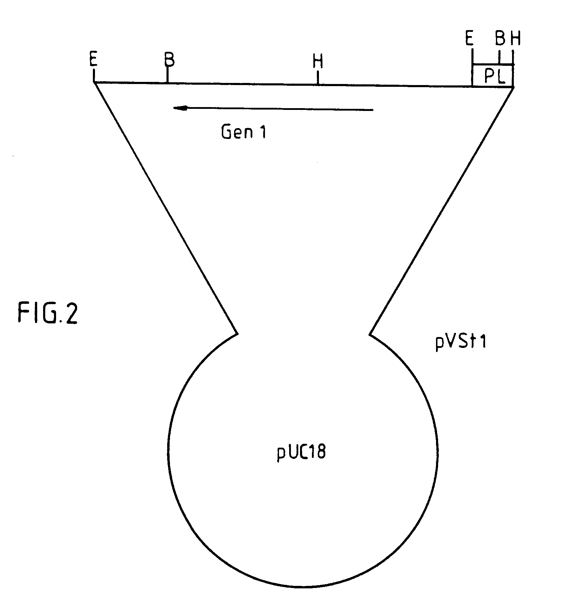

Fig. 2    stellt das Plasmid pVSt1 dar, Das Gen 1 liegt auf dem ca. 4,9 kb großen EcoRI Fragment. Es kann auch durch Doppelverdau mit BamHI und EcoRI isoliert werden. HindIII schneidet im Gen. Der Pfeil gibt die Orientierung des Gens auf dem Plasmid an.

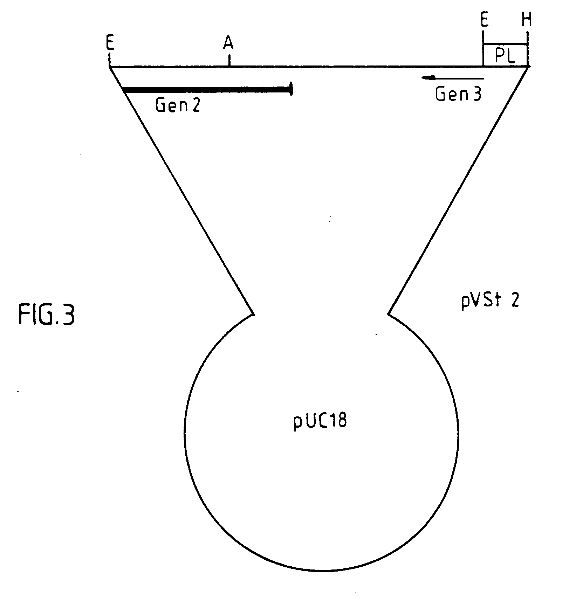

Fig. 3    stellt das Plasmid pVSt2 dar. Dieses Plasmid enthält ein 3,4 kb EcoRI Fragment von pVSt12t3, das die terminalen Bereiche von Gen 3 und proximale Bereiche von Gen 2 enthält. Durch Zirkularisation dieses Fragments entsteht ein funktionelles Stilbensynthase Gen. Dieses Gen ist ein synthetisches Gen aus Gen 2 und Gen 3.

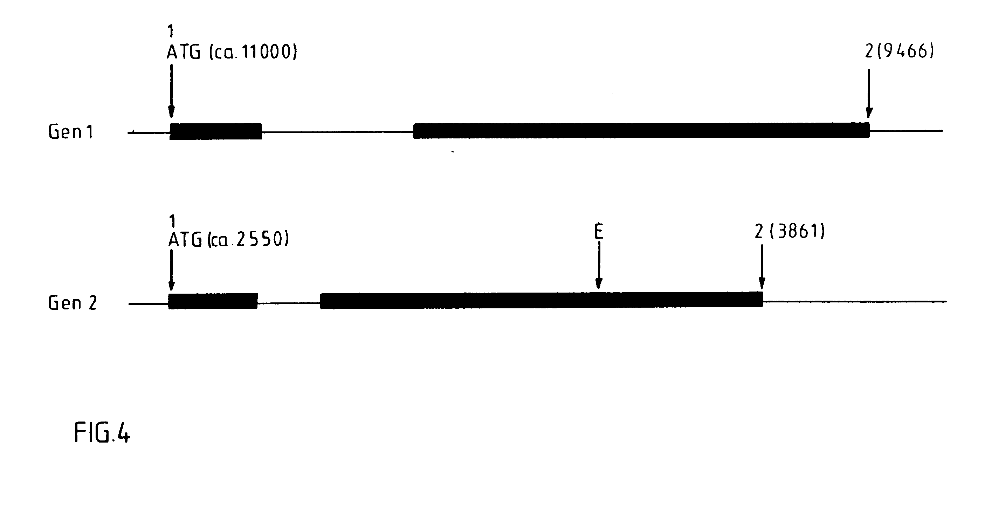

Fig. 4    stellt kodierende (Exons/fett gedruckt) und nicht kodierende Bereiche (Introns) von Gen 1 und Gen 2 dar. Die Position des Translationsinitiationskodons (ATG) liegt bei ungefähr 11.000 (s. Fig. 1) für Gen 1 und für Gen 2 bei Position 2.550. Gen 1 ist ca. 1,530 bp lang und endet bei der Position 9466. Gen 2 ist ca. 1.300 bp lang und endet bei der Position 3861. Die unterschiedliche Länge beider Gene resultiert aus unterschiedlich langen Introns.

In Fig. 1 - 4 bedeuten:

E        : EcoRI
B        : BamHI
H        : HindIII
PL       : Polylinker aus dem Plasmid pUC18
A        : Asp718
ATG      : Translationsinitiationskodon
S        : SmaI

Die erhöhte Resistenz von erfindungsgemäß transformierten Pflanzen sei anhand des folgenden Beispiels erläutert:

Beispiel A

Zur Prüfung einer erhöhten Resistenz gegenüber Pflanzenkrankheiten wurden die Pflanzen mit einem Pathogen inokuliert und der Befallsgrad als Parameter herangezogen. Als Testpathogen diente Botrytis cinerea Pers..
Die Tabakpflanzen wurden in Gewebekultur vorgezogen und anschließend im Gewächshaus in Einheitserde (Fa. Balster) in Töpfe (d= 11 cm) getopft und bei 23°C und 70-80 % rel. Luftfeuchte im Gewächshaus bis Versuchsbeginn angezogen. Die Versorgung mit Wasser und Dünger erfolgte nach Bedarf. Zur Inokulation wurden die Blätter der Pflanzen (3-4 Wochen nach Überführung ins Gewächshaus) mit Sporensuspension des Pathogens tropfnass angesprüht. Anschließend wurden die Pflanzen bei 100 % rel. Luftfeuchte und 10-20°C inkubiert. Nach 4-8 Tagen wurde der Gesundheitszustand der Pflanzen anhand der befallenden Blattfläche in Prozent ermittelt.
Wie aus den Tabellen ersichtlich wird, wiesen transformierte Tabakpflanzen, in die ein erfindungsgemäß Stilbensynthase-Gen eingesetzt wurde, einen geringeren Befall mit B. cinerea auf als die des nicht transformierten Wildtyps SR1, und zeigten somit eine erhöhte Pilz-Resistenz im Vergleich zu den nicht transformierten Kontrollpflanzen.

Tabelle

| Einfluß des Gens für Resveratrol auf den Befall der Tabakpflanzen mit Botrytis cinerea. | | | | | | | | |
|---|---|---|---|---|---|---|---|---|
| | % befallene Blattfläche auf Blatt | | | | | | | Reduktion[*] |
| | 1 | 2 | 3 | 4 | 5 | 6 | $\bar{x}$ | |
| Wildtyp (Kontrolle) | 15,0 | 16,0 | 16,5 | 11,5 | 8,6 | 2,8 | 11,7 | |
| transformierter Tabak 1 | 6,4 | 8,6 | 8,8 | 2,9 | 1,7 | 0,9 | 4,9 | 58% |
| transformierter Tabak 2 | 10,8 | 8,1 | 3,7 | 3,6 | 3,0 | 0,8 | 5,0 | 57% |

* Reduktion berechnet nach Abbott
$\bar{x}$ = Durchschnitt

**Patentansprüche**

1.  Stilbensynthase-Gene aus Wein (vitis), die dadurch gekennzeichnet sind, daß sie auf Grund der Sequenzhomologien mit den Stilbensynthase-Genen auf den Plasmiden pVSt1 (gemäß DSM 6002), pVSt2 (gemäß DSM 6003) und pVSt12t3 (gemäß DSM 6346) mit Hilfe dieser Gene aus dem Genom von Wein (vitis) nach den üblichen Methoden der Molekularbiologie erhältlich sind, einschließlich solcher Modifikationen dieser Gene, die durch den Ersatz, die Hinzufügung und/oder die Entfernung von DNA-Abschnitten, einzelner Kodons und/oder einzelner Nukleotide erhältlich sind, und bei welchen die Funktion der Stilbensynthase-Gene nicht derartig beeinträchtigt wird, daß sie nicht mehr für Stilbensynthase kodieren, ausgenommen Stilbensynthase-Gene aus Erdnuß.

2. Stilbensynthase-Gene, gemäß Anspruch 1, wobei die Stilbensynthase Resveratrolsynthase bedeutet.

3. Stilbensynthase-Gene gemäß den Ansprüchen 1 und 2, erhältlich aus Vitis vinifera und Vitis riparia.

4. Stilbensynthase-Gene gemäß den Ansprüchen 1 bis 3, erhältlich aus Vitis vinifera der Sorte Optima.

5. Stilbensynthase-Gene, entsprechend den Stilbensynthase-Genen, welche in den Plasmiden pVSt1 (gemäß DSM 6002), pVSt2 (gemäß DSM 6003) und pVSt12t3 (gemäß DSM 6346) enthalten sind, einschließlich solcher Modifikationen dieser Gene, die durch den Ersatz, die Hinzufügung und/oder die Entfernung von DNA-Abschnitten, einzelner Kodons und/oder einzelner Nukleotide erhältlich sind, und bei welchen die Funktion der Stilbensynthase-Gene nicht derartig beeinträchtigt wird, daß sie nicht mehr für Stilbensynthase kodieren, ausgenommen Stilbensynthase-Gene aus Erdnuß.

6. Stilbensynthase-Gen 1, welches auf der Gen-Einheit 1, einem etwa 4900 Basenpaare großen Fragment liegt, welches durch eine Spaltung des Plasmids pVSt12t3 (gemäß DSM 6346) mit EcoRI erhältlich ist, und Stilbensynthase-Gen 2, welches auf der Gen-Einheit 2, einem etwa 3400 Basenpaare großen Fragment liegt, welches durch eine partielle Spaltung des Plasmids pVSt12t3 (gemäß DSM 6346) mit EcoRI erhältlich ist.

7. Stilbensynthase-Gen, welches auf der Gen-Einheit liegt, welche durch die Fusion der Gen-Einheit 2 des Plasmids pVSt12t3 ( gemäß DSM 6346 ) an der EcoRI-Stelle bei 3600 Basenpaaren mit der EcoRI-Stelle bei 200 Basenpaaren des Plasmids pVSt12t3 (gemäß DSM 6346), erhältlich ist.

8. Promotor der Stilbensynthase-Gene gemäß den Ansprüchen 1 bis 7, erhältlich nach den üblichen Verfahren der Molekularbiologie aus den Stilbensynthase-Genen gemäß den Ansprüchen 1 bis 7.

9. Für Stilbensynthase kodierende DNA-Region der Stilbensynthase-Gene gemäß den Ansprüchen 1 bis 7, erhältlich nach den üblichen Methoden der Molekularbiologie aus den Stilbensynthase-Genen gemäß den Ansprüchen 1 bis 7.

10. cDNA der Stilben-Synthase-Gene gemäß den Ansprüchen 1 bis 7, erhältlich nach den üblichen Methoden der Molekularbiologie aus den Stilbensynthase-Genen gemäß den Ansprüchen 1 bis 7.

11. Vektoren, welche ein oder mehrere Stilbensynthase-Gene gemäß den Ansprüchen 1 bis 7, eine kodierende DNA-Region der Stilbensynthase-Gene gemäß den Anprüchen 9 und 10 und/oder einen Promotor gemäß Anspruch 8 enthalten.

12. Vektor-Plasmide pVSt12t3 (gemäß DSM 6346), pVSt1 (gemäß DSM 6002) und pVSt2 (gemäß DSM 6003).

13. Transformierte Mikroorganismen, welche ein oder mehrere Stilbensynthase-Gene gemäß den Ansprüchen 1 bis 7, eine kodierende DNA-Region der Stilbensynthase-Gene gemäß den Anprüchen 9 und 10 und/oder einen Promotor gemäß Anspruch 8 enthalten.

14. Escherichia coli Stämme E. coli Fier 1 pVSt1 (gemäß DSM 6002), E. coli Fier 2 pVSt2 (gemäß DSM 6003) und E. coli Fier pVSt12t3 (gemäß DSM 6346) sowie ihre Mutanten, die die gleichen Eigenschaften besitzen.

15. Verwendung der Stilbensynthase-Gene gemäß den Ansprüchen 1 bis 7, des Promotors gemäß Anspruch 8, der kodierenden DNA-Region gemäß Anspruch 9, der cDNA gemäß Anspruch 10, der Vektoren gemäß den Ansprüchen 11 und 12 und/oder der transformierten Mikroorganismen gemäß den Patentansprüchen 13 und 14 zur Transformation von Pflanzenzellen (einschließlich Protoplasten) und Pflanzen (einschließlich Pflanzenteilen und Samen).

16. Transformierte Pflanzenzellen (einschließlich Protoplasten) und Pflanzen (einschließlich Pflanzenteilen und Samen), welche ein oder mehrere Stilbensynthase-Gene gemäß den Ansprüchen 1 bis 7, Promotoren gemäß Anspruch 8, kodierende DNA-Regionen gemäß Anspruch 9 und/oder cDNA gemäß Anspruch 10 als "fremde" oder "zusätzliche" DNA enthalten.

17. Verfahren zur Herstellung transformierter Pflanzenzellen (einschließlich Protoplasten) und Pflanzen (einschließlich Pflanzenteilen und Samen), mit erhöhter Resistenz gegen Schädlinge, dadurch gekennzeichnet, daß man

(a) ein oder mehrere Stilbensynthase-Gene gemäß den Ansprüchen 1 bis 7, kodierende DNA-Regionen gemäß Anspruch 9 und/oder cDNA gemäß Anspruch 10 in den Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls vermehrt und gegebenenfalls

(c) von den so erhaltenen transformierten Pflanzen der Elterngeneration oder weiterer daraus gewonnener Generationen die gewünschten Pflanzenteile (einschließlich Samen) gewinnt.

18. Verwendung der transformierten Pflanzenzellen (einschließlich Protoplasten) und Pflanzen (einschließlich Pflanzenteilen und Samen), gemäß Anspruch 16 zur Erzeugung von Vermehrungsmaterial sowie zur Erzeugung neuer Pflanzen (einschließlich Pflanzenteilen und Samen), welche die Stilbensynthase-Gene gemäß den Ansprüchen 1 bis 7, kodierende DNA-Regionen gemäß Anspruch 9 und/oder cDNA gemäß Anspruch 10 enthalten, und deren Vermehrungsmaterial.

19. Vermehrungsmaterial, erhältlich durch die Vermehrung der transformierten Pflanzenzellen und Pflanzen gemäß Anspruch 16.

20. Transformierte Pflanzenzellen (einschließlich Protoplasten) und Pflanzen (einschließlich Pflanzenteile und Samen) gemäß Anspruch 16 , das Verfahren zu ihrer Herstellung gemäß Anspruch 17 und ihre Verwendung gemäß Anspruch 18 sowie das Vermehrungsmaterial gemäß Anspruch 19, wobei die Pflanzenzellen und Pflanzen Tabak- oder Kartoffelpflanzenzellen und Tabak- oder Kartoffelpflanzen sind.

**Claims**

1. Stilbene synthase genes from grapevine (vitis), characterized in that they are obtainable based on the sequence homologies with the stilbene synthase genes on plasmids pVSt1 (according to DSM 6002), pVSt2 (according to DSM 6003) and pVSt12t3 (according to DSM 6346) with the aid of these genes from the genome of grapevine (vitis) by the customary methods of molecular biology, including those modifications of these genes which are obtainable by replacing, adding and/or removing DNA sequences, individual codons and/or individual nucleotides, and where the function of the stilbene synthase genes is not affected in such a way that they no longer encode stilbene synthase, except for stilbene synthase genes from peanut.

2. Stilbene synthase genes according to Claim 1, where the stilbene synthase denotes resveratrol synthase.

3. Stilbene synthase genes according to Claims 1 and 2, obtainable from Vitis vinifera and Vitis riparia.

4. Stilbene synthase genes according to Claims 1 to 3, obtainable from Vitis vinifera cv. Optima.

5. Stilbene synthase genes, corresponding to the stilbene synthase genes contained in plasmids pVSt1 (according to DSM 6002), pVSt2 (according to DSM 6003) and pVSt12t3 (according to DSM 6346), including those modifications of these genes which are obtainable by replacing, adding and/or removing DNA sequences, individual codons and/or individual nucleotides, and in which the function of the stilbene synthase genes is not affected such that they no longer encode stilbene synthase, except for stilbene synthase genes from peanut.

6. Stilbene synthase gene 1, which is located on gene unit 1, a fragment of approximately 4900 base pairs in size, which can be obtained by cleaving plasmid pVSt12t3 (according to DSM 6346) with EcoRI, and stilbene synthase gene 2, which is located on gene unit 2, a fragment approximately 3400 base pairs in size, which can be obtained by partial cleavage of plasmid pVSt12t3 (according to DSM 6346) with EcoRI.

7. Stilbene synthase gene which is located on the gene unit which can be obtained by fusing gene unit 2 of plasmid pVSt12t3 (according to DSM 6346) at the EcoRI site at 3600 base pairs with the EcoRI site at 200 base pairs of plasmid pVSt12t3 (according to DSM 6346).

8. Promoter of the stilbene synthase genes according to Claims 1 to 7, obtainable by the customary processes of molecular biology from the stilbene synthase genes according to Claims 1 to 7.

9. DNA region, encoding stilbene synthase, of the stilbene synthase genes according to Claims 1 to 7, obtainable by the customary methods of molecular biology from the stilbene synthase genes according to Claims 1 to 7.

10. DNA of the stilbene synthase genes according to Claims 1 to 7, obtainable by the customary methods of molecular biology from the stilbene synthase genes according to Claims 1 to 7.

11. Vectors which contain one or more stilbene synthase genes according to Claims 1 to 7, an encoding DNA region of the stilbene synthase genes according to Claims 9 and 10 and/or a promoter according to Claim 8.

12. Vector plasmids pVSt12t3 (according to DSM 6346), pVSt1 (according to DSM 6002) and pVSt2 (according to DSM 6003).

13. Transformed microorganisms which contain one or more stilbene synthase genes according to Claims 1 to 7, an encoding DNA region of the stilbene synthase genes according to Claims 9 and 10 and/or a promoter according to Claim 8.

14. Escherichia coil strains E. coli Fier 1 pVSt1 (according to DSM 6002), E. coli Fier 2 pVSt2 (according to DSM 6003) and E. coli Fier pVSt12t3 (according to DSM 6346), as well as their mutants which have the same properties.

15. Use of the stilbene synthase genes according to Claims 1 to 7, the promotor according to Claim 8, the encoding DNA region according to Claim 9, the cDNA according to Claim 10, the vectors according to Claims 11 and 12 and/or the transformed microorganisms according to Patent Claims 13 and 14 for transforming plant cells (including protoplasts) and plants (including parts of plants, and seeds).

16. Transformed plant cells (including protoplasts) and plants (including parts of plants, and seeds) which contain one or more stilbene synthase genes according to Claims 1 to 7, promoters according to Claim 8, encoding DNA regions according to Claim 9 and/or cDNA according to Claim 10 as "foreign" or "additional" DNA.

17. Process for the production of transformed plant cells (including protoplasts) and plants (including parts of plants, and seeds) with increased resistance to pests, characterized in that

(a) one or more stilbene synthase genes according to Claims 1 to 7, encoding DNA regions according to Claim 9 and/or cDNA according to Claim 10 are introduced into the genome of plant cells (including protoplasts) and, if appropriate,

(b) complete transformed plants are regenerated from the transformed plant cells (including protoplasts) and, if appropriate, propagated, and, if appropriate,

(c) the desired parts of the plants (including seeds) are obtained from the resulting transformed plants of the parent generation or a further generation derived therefrom.

18. Use of the transformed plant cells (including protoplasts) and plants (including parts of plants, and seeds) according to Claim 16 for the production of propagation material and for the production of novel plants (including parts of plants, and seeds) which contain the stilbene synthase genes according to Claims 1 to 7, encoding DNA regions according to Claim 9 and/or cDNA according to Claim 10 and their propagation material.

19. Propagation material, obtainable by propagating the transformed plant cells and plants according to Claim 16.

20. Transformed plant cells (including protoplasts) and plants (including parts of plants, and seeds) according to Claim 16, the process for their production according to Claim 17, and their use according to Claim 18, as well as the propagation material according to Claim 19, the plant cells and plants being tobacco plant cells or potato plant cells and tobacco plants or potato plants.

**Revendications**

1. Gènes stilbène synthase de la vigne (Vitis), qui sont caractérisés en ce qu'ils sont obtenus grâce à l'homologie de séquence avec les gènes stilbène synthase des plasmides pVSt1 (d'après DSM 6002), pVSt2 (d'après DSM 6003) et pVst12t3 (d'après DSM 6346), au moyen du gène du génome de la vigne (Vitis) d'après les procédés usuels de la biologie moléculaire, y compris les modifications de ces gènes qui sont obtenues par remplacement, addition et/ou élimination de fragments d'ADN, codons isolés et/ou nucléotides isolés, et pour lesquels la fonction du gène stilbène synthase n'est pas détériorée de sorte qu'il ne code plus la stilbène synthase, à l'exclusion du gène stilbène synthase d'arachide.

2. Gènes stilbène synthase suivant la revendication 1, où la stilbène synthase représente le resvératrol synthase.

3. Gènes stilbène synthase suivant les revendications 1 et 2, obtenus de *Vitis vinifera* et de *Vitis riparia*.

4. Gènes stilbène synthase suivant les revendications 1 à 3, obtenus de *Vitis vinifera* de la variété Optima.

5. Gènes stilbène synthase, correspondant aux gènes stilbène synthase qui sont contenus dans les plasmides pVSt1 (d'après DSM 6002), pVSt2 (d'après DSM 6003) et pVst12t3 (d'après DSM 6346), y compris les modifications de ces gènes qui sont obtenues par remplacement, addition et/ou élimination de fragments d'ADN, codons isolés et/ou nucléotides isolés, et pour lesquels la fonction du gène stilbène synthase n'est pas détériorée de sorte qu'il ne code plus la stilbène synthase, à l'exclusion du gène stilbène synthase d'arachide.

6. Gène stilbène synthase 1, qui se trouve sur l'unité génique 1, sur un fragment EcoRI grand d'environ 4900 paires de bases que l'on peut obtenir par clivage du plasmide pVSt12t3 (d'après DSM 6346) par EcoRI, et gène stilbène synthase 2, qui se trouve sur l'unité génique 2, sur un fragment EcoRI grand d'environ 3400 paires de bases que l'on peut obtenir par clivage partiel du plasmide pVSt12t3 (d'après DSM 6346) par EcoRI.

7. Gène stilbène synthase, qui se trouve sur l'unité génique que l'on obtient par la fusion de l'unité génique 2 du plasmide pVSt12t3 (d'après DSM 6346) au site EcoRI, à 3600 paires de bases avec le site EcoRI à 200 paires de bases du plasmide pVSt12t3 (d'après DSM 6346).

8. Promoteur des gènes stilbène synthase suivant les revendications 1 à 7, obtenu par les procédé usuels de la biologie moléculaire à partir des gènes stilbène synthase suivant les revendications 1 à 7.

9. Région d'ADN codant la stilbène synthase, des gènes stilbène synthase suivant les revendications 1 à 7, obtenue par les procédé usuels de la biologie moléculaire à partir des gènes stilbène synthase suivant les revendications 1 à 7.

10. ADNc des gènes stilbène synthase suivant les revendications 1 à 7, obtenu par les procédé usuels de la biologie moléculaire à partir des gènes stilbène synthase suivant les revendications 1 à 7.

11. Vecteurs qui contiennent un ou plusieurs gènes stilbène synthase suivant les revendications 1 à 7, une région d'ADN codant les gènes stilbène synthase suivant les revendications 9 et 10 et/ou un promoteur suivant la revendication 8.

12. Plasmide vecteur pVSt12t3 (d'après DSM 6346), pVSt1 (d'après DSM 6002) et pVSt2 (d'après DSM 6003).

13. Microorganismes transformés qui contiennent un ou plusieurs gènes stilbène synthase suivant les revendications 1 à 7, une région d'ADN codant les gènes stilbène synthase suivant les revendications 9 et 10 et/ou un promoteur suivant la revendication 8.

14. *Escherichia coli* souche *E. coli* Fier 1 pVSt1 (d'après DSM 6002), *E. coli* Fier 2 pVSt2 (d'après DSM 6003) et *E. coli* Fier pVst12t3 (d'après DSM 6346), ainsi que leurs mutants qui possèdent les mêmes propriétés.

15. Utilisation des gènes stilbène synthase suivant les revendications 1 à 7, du promoteur suivant la revendication 8, de la région d'ADN codante suivant la revendication 9, de l'ADNc suivant la revendication 10, du vecteur suivant les revendications 11 et 12 et/ou des microorganismes transformés suivant les revendications 13 et 14 pour transformer des cellules végétales (y compris protoplastes) et des plantes (y compris parties de plante et graines).

16. Cellules végétales (y compris protoplastes) et plantes (y compris parties de plante et graines) transformées, qui contiennent un ou plusieurs gènes stilbène synthase suivant les revendications 1 à 7, des promoteurs suivant la revendication 8, des régions d'ADN codantes suivant la revendication 9 et/ou de l'ADNc suivant la revendication 10, en tant qu'ADN 〈 étranger 〉 ou 〈 supplémentaire 〉.

**17.** Procédé de préparation de cellules végétales (y compris protoplastes) et des plantes (y compris parties de plante et graines) transformées, avec une résistance accrue vis-à-vis des parasites, caractérisées en ce que l'on :

(a) introduit un ou plusieurs gènes stilbène synthase suivant les revendications 1 à 7, régions d'ADN codantes suivant la revendication 9 et/ou ADNc suivant la revendication 10 dans le génome de cellules végétales (y compris protoplastes) et facultativement,

(b) régénère à partir des cellules végétales transformées (y compris protoplastes), des plantes transformées complètes et facultativement les multiplie, et facultativement

(c) obtient des plantes transformées ainsi obtenues, de la génération parent ou d'autres générations produites à partir de celle-ci, les parties de plantes souhaitées (y compris les graines).

**18.** Utilisation des cellules végétales (y compris protoplastes) et des plantes (y compris parties de plante et graines) transformées suivant la revendication 16, pour produire un matériau de multiplication ainsi que pour produire de nouvelles plantes (y compris parties de plantes et graines), qui contiennent les gènes stilbène synthase suivant les revendications 1 à 7, des régions d'ADN codantes suivant la revendication 9 et/ou de l'ADNc suivant la revendication 10, et leur matériau de multiplication.

**19.** Matériau de multiplication, obtenu par multiplication des cellules végétales et plantes transformées suivant la revendication 16.

**20.** Cellules végétales (y compris protoplastes) et des plantes (y compris parties de plante et graines) transformées suivant la revendication 16, le procédé pour leur préparation suivant la revendication 17 et leur utilisation suivant la revendication 18, ainsi que le matériau de multiplication suivant la revendication 19, où les cellules végétales et les plantes sont des cellules végétales de tabac ou de pomme de terre et des plantes de tabac ou de pomme de terre.

FIG.1

FIG.2

FIG. 3

FIG.4